# EUROPEAN PATENT APPLICATION

(11) **EP 1 468 606 A1**
(43) Date of publication of application: **20.10.2004**
(21) Application number: 04075863.3
(22) Date of filing: 18.03.2004
(51) Int. Cl.: A01N 31/02, A01N 31/04, A01N 31/14, A61L 2/18

(54) **Process for the chemical and thermal disinfection**

(30) Priority: 17.04.2003 DE 10317932
(71) Applicant: Air Liquide Santé (International), 75341 Paris Cedex 07 (FR); SCHÜLKE & MAYR GMBH, 22851 Norderstedt (DE)
(72) Inventor: Beilfuss, Wolfgang, 22339 Hamburg (DE); Gradtke, Ralf, 25436 Tornesch (DE); Mohr, Michael, 24568 Kaltenkirchen (DE); Goroncy-Bermes, Peter, 22145 Hamburg (DE); Behrends, Sabine, 25482 Appen (DE)
(74) Representative: Conan, Philippe Claude

(57) **Abstract**

Use of a composition which contains (a) one or more 1-or 2-(C₃ to C₂₄-alkyl)glycerol ethers and (b) one or more aromatic alcohols for the disinfection of the surface of an article at a temperature above 25°C.

## Description

The present invention relates to the use of a composition which contains (a) glycerol ether and (b) aromatic alcohol for thermochemical disinfection.

Mechanical disinfection methods are employed for disinfecting instruments especially in the clinical sector. In these, an aqueous composition is allowed to act, normally at elevated temperature, on the surface to be disinfected, cf. Anforderungen an die Hygiene bei der Aufbereitung bei Medizinprodukten, Bundesgesundheitsblatt 44 (2001), 1115-1126.

Preparations currently employed in thermochemical disinfection methods can be roughly divided into three groups:

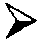 Compositions which contain short-chain organic acids such as formic acid, acetic acid or citric acid.

The effect of such monobasic or polybasic acids is disclosed inter alia in EP-A-0 505 763 and AT-A-382 310, see also Hygiene + Medizin 1989, 14, pages 69 et seq., GB-A-2 103 089 and Tierärztliche Umschau 1988, 43, pages 646 et seq. It is additionally proposed in DE-C2-42 00 066 to employ a 1.5% by weight aqueous solution of citric acid, optionally with the addition of malic acid or lactic acid, to inactivate hepatitis B viruses. However, a disadvantage which has emerged is that such disinfectants necessarily have a low pH and accordingly, especially when relatively high temperatures are used for disinfecting instruments, act as strong corrosives.

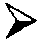 Compositions which contain quaternary ammonium compounds.

These have proved, especially in disinfectants with a very high alcohol content, e.g. in anhydrous isopropanol/n-propanol or 80% strength ethanol, to be effective hand disinfectants (see, inter alia, Wallhäußer, Praxis der Sterilisation, Henkel Chemische Bibliothek, 4th edition, 1988, pages 75 et seq.). Disinfectants with a high alcohol content are, however, unsuitable for disinfecting instruments because they attack synthetic materials. In addition, disinfectants containing quaternary ammonium compounds are highly foaming, which restricts their use, especially for disinfecting instruments.

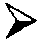 Compositions which contain aldehydes such as formaldehyde, acetaldehyde and glutaraldehyde.

Aldehyde-containing disinfectants have been unwanted for some years because of the harmful effects on human health, especially of formalin, and because of their unpleasant odour.

Many materials (e.g. metal) of the surfaces of instruments (e.g. endoscopes) are moreover resistant to the agents used in known compositions for thermochemical disinfection for only a limited time and in a limited temperature range. Thus, known solutions used for thermochemical disinfection lead, at the temperatures necessary for use, to corrosion of the surfaces of treated articles, e.g. to rusting of metal, to hazing of glass, plastic or ceramics or to brittle synthetic materials. Replacement of the agents, lowering the concentration used and/or lowering the disinfection temperature are, however, subject to restrictions because the surface must be dependably cleaned and reliably disinfected and moreover a large number of microorganisms must be eliminated.

The disinfectant solutions conventionally employed, and corresponding concentrates, have disadvantages, however:
1. Many known agents are costly, leading to the respective disinfection method being uneconomic, especially if it is necessary to use high concentrations.
2. The known solutions for use must (i) contain comparatively high concentrations of agents, (ii) be employed at comparatively high temperatures and (iii) for a comparatively long time for dependable elimination of all relevant microorganisms. These three parameters are not unrestrictedly consistent with material-conserving disinfection and may lead to stress for the staff and/or environment from the solutions used.
3. Disinfectant concentrates are often not stable at low temperatures or on storage and are prone to discoloration and to foaming of the solutions prepared for use by dilution with water. To preclude these disadvantages of the concentrates it is necessary to add to the concentrates auxiliaries which are likewise subject to the restrictions described for the agents.
4. Many agents require the addition of appropriate auxiliaries for it to be possible to handle the agents in solutions diluted for use (and for example to afford solutions for use which are clear in the use concentration).
5. Many agents are effective only for certain microorganisms. The formulation of disinfectants (solutions for use, concentrates) with three or more components in order to ensure efficacy for all relevant microbes leads to additional problems, however.
6. Many agents are unacceptable as residues even in small quantities, which is why careful rinsing of the disinfected surface with water is necessary. This procedure is uneconomic, time-consuming, of low environmental compatibility and may lead through the rinsing water to recontamination with unwanted microorganisms.

DE-A-40 26 756 relates to preservatives which contain as synergistic agents a mixture of a) an organic acid, b) a monophenyl glycol ether and c) a guanidine derivative. Examples 13 and 14 are concentrates with more than 60% by weight of phenoxyethanol and respectively 15 and 10% by weight of glycerol monoalkyl ether. The preservatives in DE-40 26 756 are effective for various bacteria and yeasts. The applicant's post-published DE A-102 24 979 discloses mixtures of glycerol ethers with aromatic alcohols for controlling mycobacteria. Use of the mixtures at elevated temperature is not described.

DE-A-41 40 474 relates to the use of glycerol monoalkyl ethers as refatting skincare additives. DE-A-100 25 122, DE-A-100 25 123 and DE-A-100 25 124 disclose preparations having a content of glycerol monoalkyl ether. The preparations are used for preserving cosmetic and dermatological preparations.

DE-C-42 40 674 discloses that glycerol monoalkyl ethers of the formula R-O-CH₂-CHOH-CH₂OH have a deodorant effect. In addition, a combination of 0.15% by weight of phenoxyethanol with 0.135% by weight of 1-(2-ethylhexyl)glycerol ether (proprietary name Sensiva SC 50) which additionally contains 40% by weight of ethanol and 0.015% by weight of dibromodicyanobutane is described. The compositions are used to preserve cosmetic, pharmaceutical or cleansing products. Use in a thermochemical disinfection method is not disclosed.

DE-C-41 40 473 discloses compositions which can be used as skin antiseptics and hand disinfectants and which contain a combination of an aliphatic C₁- to C₆-alkyl alcohol component and at least one glycerol monoalkyl ether in aqueous solution. A preferred glycerol ether is 1-(2-ethylhexyl)glycerol ether (Sensiva SC 50). The high content of aliphatic C₁- to C₆-alkyl alcohol component, and the corrosive effect associated therewith, especially at elevated temperature, appears to preclude use of the composition in a thermochemical disinfection method.

DE-A-41 24 664 describes mixtures having antimicrobial activity and containing a synergistic combination of aryl-substituted alkanol with diol. Exemplary diols are glycerol monoalkyl ethers. The mixtures are used to preserve aqueous preparations of substances susceptible to microbial degradation (oils, fats, proteins, carbohydrates or derivatives thereof).

The Patent DRP 649 206 of 5 August 1937 relates to a disinfection method in which a glycerol monoalkyl ether is used as aqueous solution or emulsion, for example for disinfectant treatment of equipment in the food and other consumables industries. The effect of the glycerol ethers at a temperature of 50°C on the yeast *Mycoderma* and the mould *Oidium lactis* for example was investigated. It is pointed out as advantageous to combine the microbicidal effect with the effect of known disinfectants such as hypochlorites, peroxy compounds, metals and metal salts or organic substances such as phenols. Combination of glycerol ether with alcohol is not disclosed. DRP 649 206 furthermore does not relate to activity on microbes which must be controlled in the hospital sector.

No compositions containing glycerol ethers for thermo chemical disinfection are known to be on the market. It was moreover found in tests carried out for the purposes of the present invention that glycerol ethers such as Sensiva SC 50 are particularly active above 25°C on bacteria (gram-positive and gram-negative) and mycobacteria, but the activity on yeasts (e.g. *Candida albicans)* and especially on moulds (e.g. *Aspergillus niger)* in a use concentration of 0.1% by weight still requires improvement.

The present invention was thus based on the object of providing a composition for thermochemical disinfection of surfaces of articles such as instruments and thermolabile materials, which satisfies the stated requirements and avoids the disadvantages of the state of the art. The object was in particular to provide for a thermochemical disinfection method a composition which
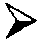 does not attack or attacks negligibly materials used in the hospital sector as surfaces of articles and which must be disinfected, also above room temperature, i.e. above 25°C, and
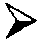 has no irritant or defatting effect on contact with human skin (that is, does not necessarily have a high content of lower alcohols such as ethanol or isopropanol).

It has now surprisingly been found according to the invention that it is possible to use a composition which contains (a) one or more 1- or 2-(C₃- to C₂₄-alkyl) glycerol ethers and (b) one or more aromatic alcohols for disinfecting the surface of an article at a temperature above room temperature (room temperature defined as 25°C), preferably a temperature of 30°C or above, more preferably 35°C or above, in particular 40°C or above, most preferably 50°C or above.

In one embodiment, the disinfection is carried out without elevated pressure. The disinfection temperature in this case is preferably 40 to 80°C, more preferably 45 to 60°C, in particular 45 to 55°C, for example about 50°C.

In a further embodiment of the invention, the disinfection is carried out under elevated pressure. The disinfection temperature in this case is up to 170°C and is preferably in the range from 80 to 160°C, more preferably 100 to 150°C, in particular 120 to 140°C, for example 130 to 135°C. At present, a maximum temperature of 134°C for a period of 20 minutes is regarded as adequate for example for the inactivation of prions, in the case of thermal inactivation.

A sterilization (eradication of viable microbes) of inanimate surfaces above 100°C can take place for example in an autoclave with superheated, saturated steam or a steam/air mixture under elevated pressure. Thus, for example, a steam sterilization or autoclaving can take place at a minimum of 120°C, corresponding to a gauge pressure of 1 bar, acting for a time of from 15 to 20 minutes with the addition of a composition according to the invention.

The use takes place on inanimate surfaces, e.g. during the thermochemical disinfection and/or cleaning of instruments, thermostable materials and thermolabile materials, such as endoscopes.

The use according to the invention can take place by wetting, spraying, rubbing, wiping or moistening the surface with the composition, dipping the surface into the composition, or disinfecting the surface by atomizing the composition. The treated surface of the article in this connection is any inorganic or organic material, in particular a thermosensitive material, for example made of metal, glass, wood, plastic, textile or ceramic. The article may be a medical instrument or laboratory apparatus, a product system or a part thereof, for example a pipeline or a storage tank, a foodstuffs container such as a bottle, a product which is subject to the Medical Appliances Act, an air-conditioning system, a membrane, an ion exchanger or a cooling water circulation. The use takes place for example in the manual and mechanical disinfection and preparation of medical instruments and appliances, especially thermolabile instruments such as flexible endoscopes.

The use referred to here as disinfection may be any cleaning, preservation, sterilization, instrument preparation, system disinfection or maintenance, but is preferably a mechanical use. The disinfection time is, for example, 10 seconds to 1 hour, more preferably 1 minute to 30 minutes, in particular 5 to 15 minutes.

The thermochemical disinfection of instruments, especially of thermolabile instruments such as flexible endoscopes, is carried out in special automatic cleaning and disinfecting systems. An example of a programme procedure according to the invention, in which the composition is advantageously employed in the form of an instrument disinfectant, is as follows:
1. where appropriate precleaning with cold water,
2. cleaning at 55 to 60°C with a neutral cleaner (e.g. as 0.5% strength solution),
3. thermochemical disinfection at 55 to 60°C acting for a time of from 1 to 20 minutes, e.g. about 5 minutes, with a disinfectant (e.g. 1 to 3% strength based on a concentrate),
4. rinsing with cold water and
5. drying.

An alternative method includes the steps of:
1) where appropriate precleaning with cold water,
2) cleaning with a neutral cleaner, raising the temperature to 90 to 100°C, preferably 90 to 95°C, such as about 93°C,
3) thermochemical disinfection at 90 to 100°C, preferably 90 to 95°C, such as about 93°C, acting for a time of from 1 to 20 minutes with the composition,
4) rinsing with water and
5) drying, where appropriate at 40 to 60°C.

The statements concerning the thermochemical disinfection according to the invention preferably relate to step 3 of the method described above.

In one embodiment of the invention, the composition used is in the form of a liquid concentrate and, for use, is diluted with water to an aqueous solution for use. Such a concentrate contains, for example, (a) 5 to 20% by weight, such as 10% by weight, of glycerol ether and (b) 70-95% by weight, such as 80 or 90% by weight, of aromatic alcohol and, where appropriate, up to 10% by weight of water. Examples of concentrates consist of (i) 10% by weight of 1-(2-ethylhexyl)glycerol ether and 90% by weight of phenoxyethanol, (ii) 10% by weight of 1-(2-ethylhexyl)glycerol ether, 80% by weight of phenoxyethanol and 10% by weight of water and (iii) 6% by weight of 1-(2-ethylhexyl)glycerol ether, 90% by weight of phenoxyethanol and 4% by weight of water. The skilled person is able to formulate appropriate water-dilutable concentrates with the assistance of suitable solvents (glycols such as propylene glycol), solubilizers, acids, alkalizing agents or surfactants.

A preferred concentrate is anhydrous. Although concentrates are normally liquid, it is possible to produce pasty or solid concentrates by suitable procedures.

In one embodiment, the composition is employed as aqueous solution for use and then contains (a) 0.01 to 1.0% by weight, more preferably 0.025 to 0.5% by weight, in particular 0.05 to 0.2% by weight, particularly preferably about 0.1% by weight of glycerol ether and (b) 0.1 to 10% by weight, more preferably 0.25 to 5% by weight, in particular 0.5 to 2% by weight of aromatic alcohol. Such a solution for use may contain 89% by weight or more, preferably 94.5 to 99.725% by weight, in particular 97.8 to 9945% by weight, of water. Solutions for use preferred in this connection have a pH of from 3 to 10.

In a further embodiment, the solution for use contains salt. For example, salt-containing compositions may make it possible to control the agent concentration via the electric conductivity. For the inactivation of prions, so-called chaotropic salts are additionally employed. Corrosion-inhibiting salts are able to improve the material compatibility of the compositions.

The alkyl group of the glycerol ether is preferably selected from branched or unbranched saturated alkyl. Preferred 1-monoalkylglycerol ethers have saturated (unbranched or, preferably, branched) C₃- to C₁₈-alkyl, particularly preferably saturated and branched C₆- to C₁₂-alkyl. Examples of 1- or 2-alkylglycerol ethers are 1- or 2-propylglycerol ether, -octylglycerol ether, -decylglycerol ether, -dodecylglycerol ether, -hexadecylglycerol ether, -octadecylglycerol ether and -octadecenylglycerol ether. 1-(2-Ethylhexyl)glycerol ether (Sensiva® SC 50) is particularly preferred.

Preferred aromatic alcohols are selected from aryloxyalkanols (glycol monoaryl ethers), oligoalkanol aryl ethers and arylalkanols.

Aryloxyalkanols employed according to the invention have the formula Ar-O-(CHR)ₙ-OH with R = independently H (for n ≥ 2) or C₁- to C₆-alkyl, where n is an integer and preferably 2 to 10, more preferably 2 to 6 and in particular 2 or 3. While the group Ar may be a nuclear-substituted aryl group, preference is given to unsubstituted aryl, e.g. phenyl or naphthyl. Examples of aryloxyalkanols employed according to the invention are phenoxyethanol and phenoxypropanols. Preferred phenoxypropanols are 1-phenoxy-2-propanol, 2-phenoxy-1-propanol or mixtures thereof, and 3-phenoxy-1-propanol. The oligoalkanol aryl ethers include, for example, phenoxy-di-, -tri- and -oligoethanol and phenoxy-di-, -tri- and -oligopropanol.

Arylalkanols employed according to the invention have the formula Ar-(CHR)ₙ-OH with R = independently H (which is preferred) or C₁- to C₆-alkyl, where n is an integer and preferably 1 to 10, more preferably 1 to 6 and in particular 1, 2, 3 or 4. While the group Ar may be a nuclear-substituted aryl group, preference is given to unsubstituted aryl, e.g. phenyl or naphthyl. Examples of arylalkanols are 3-phenyl-1-propanol, phenethyl alcohol, veratryl alcohol (3,4-dimethoxyphenylmethyl alcohol), benzyl alcohol and 2-methyl-1-phenyl-2-propanol.

In one embodiment, the ratio by weight x of component (a) to component (b) in the composition employed according to the invention is 0.15 or less, preferably 0.09 or less, more preferably 0.08 to 0.03 and in particular 0.07 to 0.04.

It is particularly preferred to use a solution for use which comprises (a) 0.05 to 0.2% by weight, such as about 0.1% by weight, of 1-(2-ethylhexyl)glycerol ether and (b) 0.5 to 2.0% by weight, such as about 0.5 or about 1.0% by weight, of phenoxyethanol and/or phenoxypropanol.

Besides components (a) and (b) according to the invention, the composition may contain further components such as (c) one or more auxiliaries. However, it preferably has a low surfactant content and contains less than 5% by weight of surfactant, more preferably less than 2% by weight, particularly preferably less than 0.5% by weight of surfactant, and is in particular free of surfactant (the percentage data are based on the concentrate).

The term auxiliaries in this connection includes further agents. Preferred further agents are aldehydes, amines, phenols, halogen compounds and carboxylic acids. Further possible auxiliaries are wetting agents, cleaning components, corrosion inhibitors, surfactants (nonionic surfactants, anionic surfactants, amphoteric surfactants), buffers, acids, alkalizing agents, perfumes, dyes, salts, indicators, markers, complexing agents and antifoams. Examples of auxiliaries are sodium chloride, o-phenylphenol, triclosan, o-phthaldialdehyde, Lonzabac 12, Lonzabac LF and sodium benzoate.

In the use according to the invention of the composition, the skilled person will choose an optimum between the parameters of the use time, concentration of the components (a) and (b) and where appropriate (c), and disinfection temperature and pressure, which is consistent with the desired disinfectant action, depending on the sensitivity of the material to be disinfected.

Use of the composition leads to elimination of bacteria (gram-positive and gram-negative), yeasts and moulds, mycobacteria and viruses, for example propionibacteria (*Propionibacterium acnes*), dandruff-causing microbes (*Malassezia furfur*), prions, enveloped and/or non-enveloped viruses, odour-causing microorganisms, lower harmful organisms, protozoa and spores. Mention should be made in particular of the activity, demonstrated in the examples, of the compositions employed according to the invention on fungi, since the use of glycerol ether alone required an improvement at the concentrations acceptable for compositions for thermochemical disinfection.

In the use according to the invention, the compositions in the form of concentrates have the following advantages:
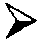 They can be formulated as liquids and with a high agent content and thus have advantages in terms of handling and cost which would not apply to a concentrate with a high water content.
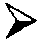 The concentrates show good colour stability.
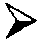 The concentrates show a broad spectrum of action even when used in low concentration.
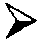 The concentrates are stable at low temperature and are also liquid and pumpable (even at -5°C).
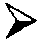 The compulsory components (a) and (b) are miscible in wide ranges with one another and are compatible with a wide range of auxiliaries (including other agents).
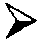 Glycerol ethers act as wetting agents and assist the disinfectant action also at corners and edges of the surface of the article.
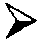 Glycerol ethers such as Sensiva SC50 are substances which have been thoroughly investigated toxicologically and have good compatibility with materials.
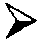 Glycerol ethers have a high boiling and flashpoint, and thus handling the concentrates poses few problems.

The aqueous solutions for use are:
clear, colourless,
low-foaming,
odour- and pH-neutral,
effectively wetting and
oxidation-, pH- and temperature-stable.

The advantages of the present invention are also evident from the following examples:

### Examples

Materials used:
SC 50 = 1-(2-Ethylhexyl)glycerol ether, Sensiva SC 50
POE = Phenoxyethanol
Water = Deionized water

All percentage data are, unless otherwise indicated, in % by weight.

### Test 1: Activity of disinfectants on bacteria and yeasts

Reduction factors were obtained with various solutions for use (SA = *Staphylococcus aureus,* PA = *Pseudomonas aeruginosa,* EC = *Escherichia coli,* CA = *Candida albicans,* AN = *Aspergillus niger,* MT = *Mycobacterium terrae*), initial microbe count 0.8-5 × 10⁹/ml, for CA 2 × 10⁷/ml, neutralizer Tryp-NaCl-TLSH (No. 22). Concerning the activity on *Mycobacterium terrae,* cf.

### Test 2.

### Method:

0.1 ml of the microbe suspension in CSL is thoroughly mixed at room temperature with 10 ml of the disinfectant dilution to be tested (in water of standardized hardness, WSH). After acting for times of 5, 15, 30 and 60 minutes, 1 ml samples of the disinfectant/microbe mixture are taken and transferred into 9 ml of inactivation liquid (0.1% tryptone + 0.85% NaCl in double-distilled water + inactivators). After a contact time not exceeding 30 minutes in the inactivation liquid, dilutions (10⁻² and 10⁻⁴ in 0.1% tryptone + 0.85% NaCl in double-distilled water) are made up. 0.1 ml samples of the inactivation liquid and the two dilutions are then transferred by spatula onto 3 CSA plates for each. As a control, the respective test microbe suspension is mixed with 10 ml of WSH in place of disinfectant. Subcultures are to be set up from this batch in the same way in parallel with the corresponding action times.

All subcultures are incubated at 37°C for 48 hours, in the case of Candida albicans at 37°C for 72 hours, and the colonies are counted. The reduction is calculated as follows: CFU between 20 and 300 per CSA plate are to be evaluated. After determination of the arithmetic mean of three values, the disinfectant effect (GRₜ) per unit time is calculated from the formula GRₜ = log_{CFU(co)} minus log_{CFU(D)} in which CFU(co) is the number of CFU per ml without exposure to the product, and CFU(D) is the number of CFU per ml after exposure to the product.

### Test 2: Activity of disinfectants on Mycobacterium terrae at room temperature

Various aqueous disinfectants were tested for their activity on *Mycobacterium terrae,* microbe count 1 to 3 × 10⁹ in a quantitative suspension test without stress. The Mycobacterium terrae (ATCC15755) quantitative suspension test of the Deutsche Gesellschaft für Hygiene und Mikrobiologie of 30 April 1997 was used (Hyg. Med. 22, 1997, No. 6, pages 278 et seq.). The following reduction factors were measured in this, with a reduction factor of > 5 corresponding to adequate activity. The aqueous solutions for use were tested after acting for a defined period. Because of the great structural similarity of *Mycobacterium terrae* with *Mycobacterium tuberculosis,* the results of the activity tests with *Mycobacterium terrae* also provide information on the activity for *Mycobacterium tuberculosis.*

### Example 1 (comparative)

### Activity of phenoxyethanol on bacteria, fungi and mycobacteria at room temperature, 30°C, 40°C and 50°C in a quantitative suspension test

The activity of phenoxyethanol (as dilution in deionized water) was investigated. The reduction factors are indicated.

| Microbe | Con centration used (%) | Room temperature | | | 30°C | | | 40°C | | | 50°C | | |
|---|---|---|---|---|---|---|---|---|---|---|---|---|---|
| | | 15' | 30' | 60' | 15' | 30' | 60' | 15' | 30' | 60' | 15' | 30' | 60' |
| SA | 0.5 | 0.00 | 0.00 | 0.00 | 0.00 | 0.00 | 0.00 | 0.53 | 0.73 | 0.39 | 0.60 | 0.83 | 0.30 |
| | 0.25 | 0.00 | 0.00 | 0.00 | 0.00 | 0.00 | 0.00 | 0.00 | 0.00 | 0.00 | 0.58 | 0.86 | 0.37 |
| EC | 0.5 | 0.00 | 0.00 | 0.00 | 0.14 | 0.29 | 0.31 | 0.00 | 0.41 | 0.52 | 4.34 | 4.05 | 4.06 |
| | 0.25 | 0.00 | 0.00 | 0.00 | 0.18 | 0.27 | 0.38 | 0.00 | 0.00 | 0.40 | 0.78 | 0.71 | 1.20 |
| PA | 0.5 | 0.00 | 0.00 | 0.00 | 0.00 | 0.00 | 0.00 | 0.00 | 0.00 | 0.00 | 2.67 | 3.74 | 5.18 |
| | 0.25 | 0.00 | 0.00 | 0.00 | 0.00 | 0.00 | 0.00 | 0.00 | 0.00 | 0.00 | 1.11 | 1.27 | 3.49 |
| CA | 0.5 | 0.46 | 0.67 | 0.59 | 0.07 | -0.10 | 0.64 | 0.00 | 0.25 | 0.47 | 0.00 | 1.16 | 1.29 |
| | 0.25 | 0.00 | 0.00 | 0.00 | 0.00 | 0.00 | 0.56 | 0.00 | 0.24 | 0.51 | 0.00 | 0.00 | 1.02 |
| AN | 0.5 | 0.00 | -0.18 | -0.08 | 0.78 | 0.70 | 0.30 | | | | 0.00 | 0.60 | 0.48 |
| | 0.25 | 0.30 | -0.10 | 0.10 | 0.48 | 0.70 | 0.60 | | | | -0.30 | -0.24 | -0.12 |
| MT | 0.5 | 0.00 | 0.00 | 0.00 | 0.00 | 0.00 | 0.00 | 0.00 | 0.00 | 0.00 | 0.00 | 0.00 | 0.00 |

### Result:

Phenoxyethanol in a concentration of 0.5% by weight is active only on gram-negative bacteria even at a temperature of 50°C and acting for a time of 60 minutes.

### Example 2

### Activity of glycerol monoalkyl ether SC 50 on bacteria, fungi and mycobacteria at room temperature, 30°C, 40°C and 50°C in a quantitative suspension test

The activity of SC 50 was investigated. The reduction factors are indicated:

| Microbe | Concentration used (%) | Room temperature | | | 30°C | | | 40°C | | | 50°C | | |
|---|---|---|---|---|---|---|---|---|---|---|---|---|---|
| | | 15' | 30' | 60' | 15' | 30' | 60' | 15' | 30' | 60' | 15' | 30' | 60' |
| SA | 0.1 | 0.00 | 0.00 | 0.96 | 0.78 | 0.53 | 0.35 | 0.97 | 1.56 | 1.45 | 2.44 | 4.93 | 4.70 |
| | 0.05 | 0.00 | 0.00 | 0.00 | 0.00 | 0.00 | 0.00 | 0.70 | 0.88 | 0.57 | 0.58 | 0.81 | 0.29 |
| EC | 0.1 | 0.00 | 0.54 | 0.50 | 0.54 | 0.58 | 1.15 | 1.84 | 3.27 | 3.46 | 3.74 | 4.95 | 4.90 |
| | 0.05 | 0.00 | 0.43 | 0.25 | 0.13 | 0.25 | 0.36 | 0.00 | 0.00 | 0.00 | 1.17 | 1.70 | 2.85 |
| PA | 0.1 | 0.00 | 0.00 | 0.00 | 0.00 | 0.00 | 1.01 | 1.03 | 1.10 | 1.14 | 3.55 | 3.50 | 4.00 |
| | 0.05 | 0.00 | 0.00 | 0.00 | 0.00 | 0.00 | 0.00 | 0.00 | 0.00 | 0.00 | 0.00 | 0.85 | 0.96 |
| CA | 0.1 | 0.00 | 0.00 | 0.00 | 0.10 | -0.08 | 0.64 | 0.55 | 0.46 | 0.65 | 1.07 | 1.53 | 4.20 |
| | 0.05 | 0.00 | 0.00 | 0.00 | 0.00 | 0.00 | 0.00 | 0.54 | 0.45 | 0.59 | 0.00 | 0.00 | 1.08 |
| AN | 0.1 | -0.35 | -0.10 | 0.40 | 0.30 | 0.70 | 0.60 | | | | -0.10 | 0.30 | 0.18 |
| | 0.05 | 0.30 | -0.10 | 0.22 | 0.08 | 0.70 | 0.60 | | | | 0.12 | 0.12 | -0.12 |
| MT | 0.1 | 0.00 | 0.00 | 0.00 | 0.00 | 0.00 | 0.00 | 0.00 | 0.00 | 0.00 | 3.10 | 3.34 | 3.48 |

### Result:

As a 0.1% by weight solution SC 50 has broad activity on gram-positive and gram-negative bacteria, yeasts and mycobacteria at 50°C. At low temperature glycerol ether in this low concentration has virtually no antimicrobial activity.

### Example 3

### Activity of a solution according to the invention on bacteria, fungi and mycobacteria at room temperature, 30°C, 40°C and 50°C in a quantitative suspension test

The activity of a mixture of 0.1% by weight SC 50 and 0.5% by weight phenoxyethanol in water ("Sol.") was investigated (also as 50% dilution). The reduction factors are indicated below.

| Microbe | Concentration used (%) | Room temperature | | | 30°C | | | 40°C | | | 50°C | | |
|---|---|---|---|---|---|---|---|---|---|---|---|---|---|
| | | 15' | 30' | 60' | 15' | 30' | 60' | 15' | 30' | 60' | 15' | 30' | 60' |
| SA | Sol. | 2.74 | 3.16 | 3.24 | 3.03 | 3.46 | 4.18 | 2.40 | 3.38 | 4.85 | 2.64 | 3.03 | 3.15 |
| | 50% | 0.00 | 0.00 | 0.00 | 0.79 | 0.56 | 0.21 | 1.11 | 1.58 | 1.40 | 0.00 | 2.05 | 3.15 |
| EC | Sol. | 4.30 | 4.85 | 4.48 | 4.60 | 4.60 | 4.70 | 4.74 | 4.78 | 4.85 | 5.04 | 4.95 | 4.90 |
| | 50% | 0.00 | 0.00 | 0.00 | 2.29 | 2.37 | 2.58 | 0.69 | 2.08 | 3.04 | 3.68 | 4.95 | 4.90 |
| PA | Sol. | 5.71 | 5.57 | 5.52 | 5.51 | 5.51 | 5.45 | 5.38 | 5.41 | 5.43 | 5.52 | 5.20 | 5.18 |
| | 50% | 0.00 | 0.00 | 0.00 | 0.00 | 0.00 | 0.00 | 0.97 | 1.37 | 1.44 | 5.52 | 5.20 | 5.18 |
| CA | Sol. | 0.43 | 0.80 | 0.76 | 0.21 | 0.77 | 2.91 | 4.78 | 3.92 | 4.85 | 5.48 | 5.53 | 5.43 |
| | 50% | 0.00 | 0.59 | 0.52 | 0.03 | -0.14 | 0.83 | 0.51 | 0.47 | 0.59 | 1.08 | 3.48 | 5.43 |
| AN | Sol. | -0.18 | -0.10 | -0.08 | 0.48 | 0.70 | 0.60 | | | | 2.60 | 2.60 | 2.48 |
| | 50% | -0.10 | 0.30 | 0.22 | 0.48 | 0.22 | 0.60 | | | | 0.30 | 1.40 | 1.88 |
| MT | Sol. | 0.00 | 0.00 | 0.00 | 2.67 | 5.74 | 5.80 | 5.61 | 5.87 | 5.89 | 5.94 | 5.94 | 5.96 |

### Result:

A combination of 0.1% by weight SC 50 and 0.5% by weight phenoxyethanol in deionized water have a broad activity at 50°C.

### Example 4

### Activity of a solution for use for Mycobacterium terrae at 50°C

Suspension tests were carried out at 50°C. The reduction factors are indicated below:

| Experiment | | Agent content | Action time | | |
|---|---|---|---|---|---|
| | | | 15' | 30' | 60' |
| 4A | (comparative) | 0.05% SC 50 | 0 4.80 | 0 | 0 |
| 4B | " | 0.10% SC 50 | | 4.66 | 5.52 |
| 4C | " | 0.5% POE | 0 | 0 | 0 |
| 4D | " | 0.75% POE | 0 | 0 | 3.19 |
| 4E | " | 1.0% POE | 3.34 | 3.73 | 5.52 |
| 4F | " | 1.5% POE | 5.18 | 5.23 | 5.45 |
| 4G | (inventive) | 0.05% SC 50 + 0.5% POE | 3.64 | 4.25 | 5.52 |
| 4H | " | 0.05% SC 50 + 0.75% POE | 4.88 | 5.23 | 4.67 |
| 4I | " | 0.1% SC 50 + 0.5% POE | 5.18 | 5.23 | 5.45 |
| 4J | " | 0.1% SC 50 + 1.0% POE | 5.64 | 5.51 | 5.52 |
| 4K | " | 0.1% SC 50 + 1.5% POE | 5.18 | 5.23 | 5.45 |

### Result:

Although neither 0.05% by weight SC 50 nor 0.75% by weight POE, even acting for a time of 60 minutes, display any activity on *Mycobacterium terrae,* the corresponding combined solution for use is active after acting for a time of only 30 minutes.

## Claims

1. Use of a composition which contains (a) one or more 1- or 2- (C₃- to C₂₄-alkyl) glycerol ethers and (b) one or more aromatic alcohols for the disinfection of the surface of an article at a temperature above 25°C.

2. Use according to Claim 1, **characterized in that** the disinfection takes place at a temperature of 30°C or above, preferably 35°C or above, in particular 40°C or above.

3. Use according to Claim 1 or 2, **characterized in that** the disinfection takes place at 40 to 80°C, more preferably from 45 to 60°C, in particular 45 to 55°C, particularly preferably at about 50°C.

4. Use according to Claim 1 or 2, **characterized in that** the disinfection takes place at up to 170°C, preferably at 80 to 160°C, more preferably 100 to 150°C, in particular 120 to 140°C, most preferably 130 to 135°C.

5. Use according to any of the preceding claims, **characterized in that** the surface is wetted, sprayed, rubbed, wiped or moistened with the composition, the surface is dipped into the composition, or the surface is disinfected by atomizing the composition.

6. Use according to any of the preceding claims, **characterized in that** the surface is made of metal, glass, wood, plastic, textile or ceramic.

7. Use according to any of the preceding claims, **characterized in that** the article is a medical instrument or laboratory apparatus.

8. Use according to any of the preceding claims, **characterized in that** the disinfection time is 10 seconds to 1 hour, more preferably 1 minute to 30 minutes, in particular 5 to 15 minutes.

9. Use according to any of Claims 1 to 7, **characterized in that** the composition is an aqueous solution for use which contains (a) 0.01 to 1.0% by weight, more preferably 0.025 to 0.5% by weight, in particular 0.05 to 0.2, particularly preferably about 0.1 % by weight of glycerol ether and (b) 0.1 to 10% by weight, more preferably 0.25 to 5% by weight, in particular 0.5 to 2% by weight, of aromatic alcohol.

10. Use according to Claim 9, **characterized in that** the aqueous solution for use contains 89% by weight or more, more preferably 94.5 to 99.725% by weight, in particular 97.8 to 99.45% by weight, of water.

11. Use according to Claim 9 or 10, **characterized in that** the solution for use has a pH of from 3 to 10.

12. Use according to any of the preceding claims, **characterized in that** the alkyl group of the glycerol ether is selected from branched or unbranched saturated alkyl.

13. Use according to Claim 12, **characterized in that** the 1- or 2-alkylglycerol ether is selected from dodecylglycerol ether, decylglycerol ether, octylglycerol ether, propylglycerol ether, octadecylglycerol ether, hexadecylglycerol ether and octadecenylglycerol ether, with preference for 1-(2-ethylhexyl)glycerol ether.

14. Use according to any of the preceding claims, **characterized in that** the aromatic alcohol is selected from aryloxylalkanols, oligoalkanols aryl ethers and arylalkanols.

15. Use according to Claim 14, **characterized in that** the aryloxyalkanol is selected from phenoxyethanol and phenoxypropanol.

16. Use according to Claim 14, **characterized in that** the arylalkanol is selected from 3-phenyl-1-propanol, phenethyl alcohol, veratryl alcohol, benzyl alcohol and 2-methyl-1-phenyl-2-propanol.

17. Use according to Claim 14, **characterized in that** the oligoalkanol aryl ether is selected from phenoxy-di-, tri- and -oligoethanol and phenoxy-di-, tri- and -oligopropanol.

18. Use according to any of Claims 9 to 17, **characterized in that** the composition is an aqueous solution for use which contains (a) 0.05 to 0.2% by weight of 1-(2-ethylhexyl)glycerol ether and (b) 0.5 to 2% by weight of phenoxyethanol and/or phenoxypropanol.

19. Use according to any of the preceding claims, **characterized in that** the composition additionally contains (c) one or more auxiliaries.

20. Use according to any of the preceding claims, **characterized in that** the disinfection time is 10 seconds to 1 hour, more preferably 1 minute to 30 minutes, in particular 5 to 15 minutes.

21. Use according to any of the preceding claims, **characterized in that** it takes place in an instrument disinfection method which comprises the following steps:
a) where appropriate precleaning with cold water,
b) cleaning at 55 to 60°C with a neutral cleaner,
c) thermochemical disinfection with the composition at 55 to 60°C acting for a time of from 1 to 20 minutes,
d) rinsing with cold water and
e) drying.

22. Use according to any of Claims 1 to 20, **characterized in that** it takes place in an instrument disinfection method which comprises the following steps:
a) where appropriate precleaning with cold water,
b) cleaning with a neutral cleaner, raising the temperature to 90 to 100°C, preferably 90 to 95°C, in particular about 93°C,
c) thermochemical disinfection with the composition at 90 to 100°C, preferably 90 to 95°C, more preferably about 93°C, acting for a time of from 1 to 20 minutes,
d) rinsing with water and
e) drying, where appropriate at 40 to 60°C.
